## Europäisches Patentamt

## European Patent Office

## Office européen des brevets

(11) Veröffentlichungsnummer: **0 283 862 B1**

(12) **EUROPÄISCHE PATENTSCHRIFT**

(45) Veröffentlichungstag der Patentschrift:
**10.10.90**

(51) Int. Cl.⁵: **C07C 43/11**, C07C 41/42, C08G 65/32

(21) Anmeldenummer: **88103773.3**

(22) Anmeldetag: **10.03.88**

(54) **Verfahren zur Entfernung von Ethylenoxid und/oder Propylenoxid aus oberflächenaktiven Derivaten.**

(30) Priorität: **18.03.87 DE 3708813**

(43) Veröffentlichungstag der Anmeldung:
**28.09.88 Patentblatt 88/39**

(45) Bekanntmachung des Hinweises auf die Patenterteilung:
**10.10.90 Patentblatt 90/41**

(84) Benannte Vertragsstaaten:
**AT BE CH DE ES FR GB GR IT LI NL SE**

(56) Entgegenhaltungen:
**US-A- 4 143 072**
**US-A- 4 443 634**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Friedrich, Klaus, Dr., Marconistrasse 13, D-4000 Düsseldorf 13(DE)**
Erfinder: **Büttgen, Franz, Schalbruch 159, D-4010 Hilden(DE)**
Erfinder: **Herrmann, Klaus, Köpenicker Strasse 33, D-4019 Monheim(DE)**

## Beschreibung

Die Erfindung betrifft ein Verfahren zur Entfernung von Ethylenoxid und/oder Propylenoxid sowie gegebenenfalls von Dioxan aus oberflächenaktiven, durch Ethoxylierung und/oder Propoxylierung hergestellten Derivaten von organischen Verbindungen, die mindestens ein aktives Wasserstoffatom aufweisen.

Durch Ethoxylierung und/oder Propoxylierung hergestellte oberflächenaktive Derivate von Verbindungen, die mindestens ein aktives Wasserstoffatom aufweisen, können herstellungsbedingt Spuren von Ethylenoxid und/oder Propylenoxid sowie von Dioxan enthalten. Gemäß einer Empfehlung des Bundesgesundheitsamtes, veröffentlicht in Bundesgesundheitsblatt 29, 21 bis 22 (1986), soll der Restgehalt an Ethylenoxid in pharmazeutischen Grundstoffen auf weniger als 1 ppm begrenzt werden.

Aus der US-PS 4 443 634 ist ein Verfahren der eingangs genannten Art bekannt geworden, bei dem die zu reinigenden oberflächenaktiven Derivate gegebenenfalls mehrfach in eine mit einem inerten Gas gefüllte Kammer gesprüht und die zu entfernenden Verunreinigungen dampfförmig abgezogen werden.

Es wurde nun gefunden, daß man Ethylenoxid und/oder Propylenoxid sowie gegebenenfalls Dioxan auf einem wesentlich einfacher zu realisierenden Weg aus den genannten oberflächenaktiven Derivaten entfernen kann, wenn man diese Derivate einer Wasserdampfbehandlung unterwirft.

Gegenstand der Erfindung ist demnach ein Verfahren zur Entfernung von Ethylenoxid und/oder Propylenoxid sowie gegebenenfalls Dioxan aus oberflächenaktiven, durch Ethoxylierung und/oder Propoxylierung hergestellten Derivaten von organischen Verbindungen, die mindestens ein aktives Wasserstoffatom aufweisen, dadurch gekennzeichnet, daß man die oberflächenaktiven Derivate bei Temperaturen von 70 bis 130 °C, gegebenenfalls im Vakuum, mit Wasserdampf behandelt und gegebenenfalls das mit Wasserdampf behandelte Produkt nachtrocknet.

Die erfindungsgemäß zu reinigenden oberflächenaktiven Derivate stellen bekannte Substanzen dar, die über gängige Methoden der organischen Synthese zugänglich sind. Ihre Herstellung erfolgt durchweg über die Ethoxylierung und/oder Propoxylierung von organischen Verbindungen, die mindestens 1 aktives Wasserstoffatom aufweisen, wie zum Beispiel Fettsäuren, Fettsäureamiden, Fettalkoholen, normalen und gehärteten Ricinusölen, Glycerinmono- und -diestern, Alkylphenolen, Polyglykolen, Fettaminen, Fettsäurealkanolamiden und Kohlehydratestern. Typische Vertreter dieser Stoffklasse sind beispielsweise ein Anlagerungsprodukt von 9 Mol Ethylenoxid an Kokosölfettsäure, ein Anlagerungsprodukt von 2 Mol Ethylenoxid an ein Fettalkoholgemisch der Kettenlänge $C_{12-14}$, ein Anlagerungsprodukt von 3 Mol Ethylenoxid und 8 Mol Propylenoxid an ein Fettalkoholgemisch der Kettenlänge $C_{12-18}$, ein Anlagerungsprodukt von 41 Mol Ethylenoxid an Ricinusöl, ein Anlagerungsprodukt von 25 Mol Ethylenoxid an gehärtetes Ricinusöl, ein Anlagerungsprodukt von 7 Gewichtsteilen Ethylenoxid an 10 Gewichtsteile eines Palmitinsäure-/Stearinsäuremono-/Diglyceridgemisches mit einem Anteil von 40 bis 45 Gewichtsprozent Monoglycerid, ein Anlagerungsprodukt von 10 Mol Ethylenoxid an Nonylphenol, ein Anlagerungsprodukt von 7,3 Mol Ethylenoxid an Glycerin, ein Anlagerungsprodukt von 10 Mol Ethylenoxid an ein Diolgemisch, das durch Umsetzung eines 1,2-Epoxyalkangemisches der Kettenlänge $C_{12-16}$ mit Ethylenglykol erhalten wurde, ein Anlagerungsprodukt von 12 Mol Ethylenoxid an ein Fettamingemisch der Kettenlaänge $C_{10-18}$, ein Anlagerungsprodukt von 4 Mol Ethylenoxid an Kokosfettsäuremonoethanoloamid und ein Anlagerungsprodukt von 20 Mol Ethylenoxid an Sorbitanmonostearat.

In einer bevorzugten Ausführungsform der Erfindung werden die oberflächenaktiven Derivate bei 100 bis 130 °C mit Wasserdampf behandelt.

Die erfindungsgemäße Reinigung der oberflächenaktiven Derivate wird zweckmäßigerweise in der Form vorgenommen, daß man Wasserdampf in die auf 70 bis 130 °C erhitzten Produkte einbläst. Dabei kann es erforderich sein, die gereinigten Substanzen nach der Wasserdampfbehandlung zu trocknen. Hierfür gibt es im wesentlichen zwei Varianten: Bei der Durchführung der Wasserdampfbehandlung bei Normaldruck mit gesättigtem Dampf wird das Produkt im Vakuum nachgetrocknet. Bei einer Behandlung unter verringertem Druck läßt man nach Beendigung der Wasserdampfbehandlung das Produkt bei angelegtem Vakuum stehen, bis der gewünschte Wassergehalt erreicht ist.

Um den Wirkungsgrad der Wasserdampfbehandlung zu erhöhen, leitet man den Wasserdampf in die zu reinigenden oberflächenaktiven Derivate vorzugsweise in feinverteilter Form ein, z. B. über Düsenköpfe. Weiterhin ist es bevorzugt, die Wasserdampfbehandlung sowie gegebenenfalls die Nachtrocknung unter Rühren vorzunehmen.

Gemäß einer weiteren bevorzugten Ausführungsform der Erfindung leitet man in die zu reinigenden oberflächenaktiven Derivate mindestens 10 kg Dampf mit einem Druck von 4 bar pro Tonne Derivat und Stunde ein; eine Menge von 30 bis 50 kg Dampf mit einem Druck von 4 bar pro Tonne und Stunde ist bevorzugt.

Gemäß einer weiteren vorteilhaften Ausführungsform der Erfindung unterwirft man die oberflächenaktiven Derivate vor der Wasserdampfbehandlung einer neutralisierenden Behandlung. Die wirkt sich insbesondere bei ethoxylierten und/oder propoxylierten Fettsäurealkanolamiden günstig aus, da sonst stärker gefärbte Produkte erhalten werden.

Mit Hilfe des erfindungsgemäßen Verfahrens können Ethylenoxid und/oder Propylenoxid und/oder Dioxan aus den oberflächenaktiven Derivaten jeweils bis zu einem Restgehalt von ≤ 1 ppm, vorzugsweise ≤ 0,1 ppm entfern werden.

Die Erfindung wird im folgenden anhand von bevorzugten Ausführungsbeispielen näher erläutert.

## Beispiel 1

Ein Fettalkoholalkoxylat, hergestellt durch Umsetzung eines Gemisches von $C_{12}$- und $C_{14}$-Fettalkoholen (Mengenverhältnis $C_{12} : C_{14}$ 70 : 30) mit 2 Mol Ethylenoxid, wurde wie folgt gereinigt: 20.000 kg Ethoxylat wurde in einen 30 m³-Rührbehälter vorgelegt und auf 110°C erhitzt. Dem Alkoxylat wurde unter mechanischem Rühren nach Erreichen dieser Temperatur 40 kg Wasserdampf mit einem Druck von 4 bar pro Tonne vorgelegtes Produkt und Stunde über einen am Behälterboden befindlichen Verteilungsring zugeführt. Die Temperatur stieg in dieser Phase auf 120 bis 130°C. Wasserdampf, Ethylenoxid und Dioxan wurden über ein Brüdenrohr auf einen über dem Rührbehälter aufgestellten Wärmeaustauscher geführt und kondensiert. Das Destillat wurde in einen Auffangbehälter überführt. Nach einer empirisch ermittelten Zeit von 2,5 Stunden wurde der Destillationsvorgang abgebrochen. Das zurückbleibende Alkoxylat enthielt Ethylenoxid und Dioxan unterhalb der Nachweisgrenze (< 1 ppm). Es wurde bei einer Temperatur von etwa 90°C unter weiterem Rühren und gleichzeitiger Evakuierung auf ca. 100 mbar bis auf eine Restfeuchte von weniger als 0,3 Gew.-% entwässert.

Das übergegangene Destillat wurde nach der Zerstörung von Ethylenoxid und Reinigung verworfen.

## Beispiel 2

Ein Alkoxylat, hergestellt durch Umsetzung eines $C_{16}/C_{18}$-Fettalkohols (50 : 50) mit 20 Mol Ethylenoxid wurde in der in Beispiel 1 beschriebenen Weise behandelt. Die Gehalte an Ethylenoxid und Dioxan lagen nach der Behandlung unterhalb der Nachweisgrenze.

## Beispiel 3

Ein Alkoxylat, erhalten aus einem $C_{12}/C_{14}/C_{16}$-Olefinepoxid, das zunächst mit Glykol und anschließend mit 1,2 Mol Propylenoxid und danach mit 9 Mol Ethylenoxid umgesetzt wurde, wurde in der in Beispiel 1 beschriebenen Weise behandelt. Das gereinigte Produkt enthielt Ethylenoxid, Propylenoxid und Dioxan unterhalb der Nachweisgrenze.

## Patentansprüche

1. Verfahren zur Entfernung von Ethylenoxid und/oder Propylenoxid sowie gegebenenfalls von Dioxan aus oberflächenaktiven, durch Ethoxylierung und/oder Propoxylierung hergestellten Derivaten von organischen Verbindungen, die mindestens ein aktives Wasserstoffatom aufweisen, dadurch gekennzeichnet, daß man die oberflächenaktiven Derivate bei Temperaturen von 70 bis 130 °C, gegebenenfalls im Vakuum, mit Wasserdampf behandelt und gegebenenfalls das mit Wasserdampf behandelte Produkt nachtrocknet.

2. Verfahren nach Anspruch 1, dadurch gekennzeichnet, daß man oberflächenaktive Derivate von Verbindungen aus der von Fettsäuren, Fettsäureamiden, Fettalkoholen, normalen und gehärteten Ricinusölen, Glycerinmono- und -diestern, Alkylphenolen, Polyglykolen, Fettaminen, Fettsäurealkanolamiden und Kohlehydratestern gebildeten Gruppe mit Wasserdampf behandelt.

3. Verfahren nach Anspruch 2 oder 3, dadurch gekennzeichnet, daß man die oberflächenaktiven Derivate bei 100 bis 130 °C mit Wasserdampf behandelt.

4. Verfahren nach mindestens einem der Ansprüche 1 bis 3, dadurch gekennzeichnet, daß man in die oberflächenaktiven Derivate Wasserdampf in feinverteilter Form einleitet.

5. Verfahren nach mindestens einem der Ansprüche 1 bis 4, dadurch gekennzeichnet, daß man die Wasserdampfbehandlung unter Rühren vornimmt.

6. Verfahren nach mindestens einem der Ansprüche 1 bis 5, dadurch gekennzeichnet, daß man mindestens 10 kg Dampf mit einem Druck von 4 bar pro Tonne oberflächenaktives Derivat und Stunde einleitet.

7. Verfahren nach mindestens einem der Ansprüche 1 bis 6, dadurch gekennzeichent, daß man 30 bis 50 kg Dampf mit einem Druck von 4 bar pro Tonne oberflächenaktives Derivat und Stunde einleitet.

8. Verfahren nach mindestens einem der Ansprüche 1 bis 7, dadurch gekennzeichnet, daß man die oberflächenaktiven Derivate, insbesondere ethoxylierte und/oder propoxylierte Fettsäurealkanolamide, vor der Wasserdampfbehandlung neutralisiert.

9. Verfahren nach mindestens einem der Ansprüche 1 bis 8, dadurch gekennzeichnet, daß man Ethylenoxid und/oder Propylenoxid und/oder Dioxan jeweils bis zu einem Restgehalt von $\leq$ 1 ppm, insbesondere $\leq$ 0,1 ppm entfernt.

## Claims

1. A process for the removal of ethylene oxide and/or propylene oxide and, optionally, dioxane from surface-active derivatives – prepared by ethoxylation and/or propoxylation – of organic compounds containing at least one active hydrogen atom, characterized in that the surface-active derivatives are treated with steam, optionally in vacuo, at temperatures of 70 to 130°C and the steamtreated product is optionally dried.

2. A process as claimed in claim 1, characterized in that surface-active derivatives of compounds from the group consisting of fatty acids, fatty acid amides, fatty alcohols, normal and hydrogenated castor oils, glycerol mono- and diesters, alkyl phenols, polyglycols, fatty amines, fatty acid alkanolamides and carbohydrate esters are treateßd with steam.

3. A process as claimed in claim 2 or 3, characterized in that the surface-active derivatives are treated with steam at 100 to 130°C.

4. A process as claimed in at least one of claims 1 to 3, characterized in that steamis introduced into the surface active derivatives in finely divided form.

5. A process as claimed in at least one of claims 1 to 4, characterized in that the treatment with steam is carried out with stirring.

6. A process as claimed in at least one of claims 1 to 5, characterized in that at least 10 kg steam under a pressure of 4 bar are introduced per tonne surface-active derivative per hour.

7. A process as claimed in at least one of claims 1 to 6, characterized in that 30 to 50 kg steam under a pressure of 4 bar are introduced per tonne surface-active derivative per hour.

8. A process as claimed in at least one of claims 1 to 7, characterized in that the surface-active derivatives, particularly ethoxylated and/or propoxylated fatty acid alkanolamides, are neutralized before the treatment with steam.

9. A process as claimed in at least one of claims 1 to 8, characterized in that ethylene oxide and/or propylene oxide and/or dioxane are each removed to a residual content of ≤ 1 ppm and, more particularly, ≤ 0.1 ppm.

**Revedications**

1. Procédé pour l'élimination de l'oxyde d'éthylène et/ou de l'oxyde de propylène, ainsi qu'éventuellement du dioxanne, hors de dérivés tensioactifs, préparés par oxyéthylation et/ou oxypropylation, de composés organiques qui comportent au moins un atome d'hydrogène actif, caractérisé en ce que l'on traite par la vapeur d'eau les dérivés tensioactifs à des températures de 70 à 130°C, éventuellement sous vide, et qu'éventuellement on sèche ensuite le produit traité par la vapeur d'eau.

2. Procédé selon la revendication 1, caractérisé en ce que l'on traite par la vapeur d'eau des dérivés tensioactifs de composés choisis parmi des acides gras, amides d'acides gras, alcools gras, huiles de ricin normales et hydrogénées, mono- et diesters de glycérol, alkylphénols, polyglycols, amines grasses, alcanolamides d'acides gras et esters de glucides.

3. Procédé selon la revendication 2 ou 3, caractérisé en ce que l'on traite par de la vapeur d'eau les dérivés tensioactifs à 100–130°C.

4. Procédé selon l'une quelconque des revendications 1 à 3, caractérisé en ce que l'on envoie dans les dérivés tensioactifs de la vapeur d'eau sous forme finement divisée.

5. Procédé selon l'une quelconque des revendications 1 à 4, caractérisé en ce que l'on effectue le traitement par la vapeur d'eau sous agitation.

6. Procédé selon l'une quelconque des revendications 1 à 5, caractérisé en ce que l'on envoie au moins 10 kg de vapeur d'eau sous une pression de 4 bars par tonne de dérivé tensioactif et par heure.

7. Procédé selon l'une quelconque des revendications 1 à 6, caractérisé en ce que l'on envoie de 30 à 50 kg de vapeur sous une pression de 4 bars par tonne de dérivé tensioactif et par heure.

8. Procédé selon l'une quelconque des revendications 1 à 7, caractérisé en ce que, avant le traitement par la vapeur d'eau, on neutralise les dérivés tensioactifs, en particulier des alcanolamides d'acides gras oxyéthylés et/ou oxypropylés.

9. Procédé selon l'une quelconque des revendications 1 à 8, caractérisé en ce que l'on élimine l'oxyde d'éthylène et/ou l'oxyde de propylène et/ou le dioxanne chacun jusqu'à une concentration résiduelle ≤ 1 ppm, en particulier ≤ 0,1 ppm.